(19) Europäisches Patentamt
European Patent Office
Office européen des brevets

(11) **EP 1 157 072 B1**

(12) **FASCICULE DE BREVET EUROPEEN**

(45) Date de publication et mention de la délivrance du brevet:
**18.08.2004 Bulletin 2004/34**

(21) Numéro de dépôt: **00905113.7**

(22) Date de dépôt: **11.02.2000**

(51) Int Cl.[7]: **C09D 17/00**

(86) Numéro de dépôt international:
**PCT/FR2000/000342**

(87) Numéro de publication internationale:
**WO 2000/049099 (24.08.2000 Gazette 2000/34)**

(54) **SOL ORGANIQUE ET COMPOSE SOLIDE A BASE D'OXYDE DE TITANE ET D'UN COMPOSE AMPHIPHILE ET PROCEDES DE PREPARATION**

ORGANISCHE SOL UND FESTSTOFF AUF BASIS VON TITANIUMOXYD UND EINEM AMPHIPHILEN STOFF UND VERFAHREN ZUR HERSTELLUNG

ORGANIC SOL AND SOLID COMPOUND BASED ON TITANIUM OXIDE AND AN AMPHIPHILIC COMPOUND AND PREPARATION METHODS

(84) Etats contractants désignés:
**AT BE CH CY DE DK ES FI FR GB GR IE IT LI LU MC NL PT SE**

(30) Priorité: **17.02.1999 FR 9901940**

(43) Date de publication de la demande:
**28.11.2001 Bulletin 2001/48**

(73) Titulaire: **RHODIA CHIMIE**
**92408 Courbevoie Cédex (FR)**

(72) Inventeurs:
- **AUPAIX, Nicole**
  **F-78370 Plaisir (FR)**
- **CHANE-CHING, Jean-Yves**
  **F-95600 Eaubonne (FR)**

(74) Mandataire: **Dubruc, Philippe**
**Rhodia Services,**
**Direction de la Propriété Industrielle,**
**40, rue de la Haie-Coq**
**93306 Aubervilliers Cedex (FR)**

(56) Documents cités:
**WO-A-97/10185**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne un sol organique et un composé solide à base d'oxyde de titane et d'un composé amphiphile et leurs procédés de préparation.

Les sols ou dispersions colloïdales d'oxyde de titane dans des milieux organiques sont connus. Toutefois, les procédés de préparation de ces sols sont complexes du point de vue industriel. Une première classe de procédés utilise des composés sensibles à l'eau comme les alcoxydes de titane. Une autre classe passe généralement par la préparation d'un sol aqueux dans un premier temps et, dans un deuxième temps, par la mise en contact de ce sol aqueux avec une phase organique pour effectuer le transfert de l'oxyde de titane dans la phase organique. Une telle manière d'opérer n'est pas adaptée non plus à la préparation de sols dans des phases polaires miscibles à l'eau. Il existe donc un besoin pour des procédés plus simples et permettant d'accéder aussi à des sols de caractéristiques variées.

L'objet de la présente invention est de fournir de tels procédés et de tels sols organiques.

Dans ce but, le sol organique de l'invention est caractérisé en ce qu'il comprend des particules d'oxyde de titane; une phase liquide organique et au moins un composé amphiphile choisi parmi les alkyl éthers phosphates polyoxyéthylénés.

L'invention concerne aussi un procédé de préparation d'un tel sol qui, selon une première variante, est caractérisé en ce qu'on mélange le composé amphiphile précité et la phase liquide organique, puis on disperse les particules d'oxyde de titane dans le mélange obtenu. Selon une seconde variante, le procédé est caractérisé en ce qu'on forme un mélange de particules d'oxyde de titane et d'au moins un composé amphiphile précité puis on disperse dans la phase liquide organique ledit mélange.

Les sols de l'invention présentent l'avantage de pouvoir exister dans une large gamme de solvants, solvants polaires ou non polaires.

D'autres caractéristiques, détails et avantages de l'invention apparaîtront encore plus complètement à la lecture de la description qui va suivre, ainsi que des divers exemples concrets mais non limitatifs destinés à l'illustrer.

Pour la suite de la description, l'expression sol ou dispersion colloïdale d'oxyde de titane désigne tout système constitué de fines particules solides de dimensions colloïdales à base d'oxyde de titane en suspension dans une phase liquide, lesdites espèces pouvant en outre, éventuellement, contenir des quantités résiduelles d'ions liés ou adsorbés tels que par exemple des chlorures, sulfates, nitrates, acétates, citrates, ammoniums ou des bases organiques telles que la diéthylamine. On notera que dans de telles dispersions, le titane peut se trouver soit totalement sous la forme de colloïdes, soit simultanément sous la forme d'ions et sous la forme de colloïdes.

Les particules du sol selon l'invention peuvent être à base de dioxyde de titane de structure cristalline majoritairement anatase. "Majoritairement" signifie que le taux d'anatase des particules de dioxyde de titane est supérieur à 50 % en masse. De préférence, les particules présentent un taux d'anatase supérieur à 80 %. Le taux de cristallisation et la nature de la phase cristalline sont mesurés par diffraction RX.

Ces particules peuvent être aussi de structure rutile.

Le diamètre moyen de ces particules dans le sol est généralement d'au plus 250 nm, de préférence d'au moins 15 nm, encore plus préférentiellement compris entre 20 et 70 nm. On précise ici que le diamètre moyen des particules ou colloïdes doit être entendu comme désignant le diamètre hydrodynamique moyen de ces derniers, et tel que déterminé par diffusion quasi-élastique de la lumière selon la méthode décrite par Michael L. Mc CONNELL dans la revue Analytical Chemistry 53, n° 8, 1007 A, (1981).

Les particules du sol selon l'invention présentent généralement une surface spécifique BET d'au moins 200 $m^2/g$, de préférence d'au moins 250 $m^2/g$.

On entend par surface spécifique BET, la surface spécifique déterminée par adsorption d'azote conformément à la norme ASTMD 3663-78 établie à partir de la méthode BRUNAUER - EMMETT - TELLER décrite dans le périodique "The Journal of the American Society", 60, 309 (1938). Pour mesurer la surface spécifique des particules selon l'invention, lorsqu'elles se présentent sous forme de dispersion, il est essentiel de suivre le protocole de mesure qui consiste à éliminer la phase liquide de la dispersion puis à sécher les particules sous vide à une température de 150°C pendant au moins 4 heures.

Selon deux variantes de l'invention, les particules d'oxyde de titane du sol peuvent comporter un revêtement. Avant la description qui va être faite de ces deux variantes, on peut préciser ici que dans les deux cas, les particules présentent un diamètre moyen généralement d'au plus 100 nm, de préférence d'au moins 25 nm, encore plus préférentiellement comprise entre 50 et 70 nm. Ces particules revêtues présentent généralement aussi une surface spécifique BET d'au moins 70 $m^2/g$, de préférence d'au moins 100 $m^2/g$.

Dans la première de cette variante, les particules d'oxyde de titane sont recouvertes au moins partiellement d'une couche d'au moins un oxyde, hydroxyde ou oxyhydroxyde métallique ou de silicium. On pourra se référer pour cette première variante à la demande de brevet EP-A-880564 dont l'enseignement concernant la description du produit et son mode de préparation est incorporé ici. Ces oxydes, hydroxydes ou oxyhydroxydes métalliques peuvent être en

particulier choisis parmi SiO2, Zr02, les oxydes, hydroxydes ou oxyhydroxydes de l'aluminium. de zinc, de titane ou d'étain sous forme simple ou mixte. Par mixte, on entend un composé métallique à base d'au moins deux des éléments précités (silicoaluminates, ...).

En général, le rapport en poids du ou des oxydes, hydroxydes ou oxyhydroxydes métalliques sur le dioxyde de titane est d'au plus 60 % en poids. Ce rapport est fonction de l'application à laquelle les particules sont destinées. De préférence, lorsque les particules sont utilisées en application cosmétique, ce rapport est d'au plus 25 %, encore plus préférentiellement d'au plus 20 %.

Cette quantité d'oxyde, hydroxyde ou oxyhydroxyde métallique est mesurée sur les particules en dispersion par fluorescence X.

Selon un mode de réalisation particulier de l'invention, les particules sont recouvertes au moins partiellement d'une couche de silice et/ou d'un oxyde, hydroxyde ou oxyhydroxyde d'aluminium sous forme simple ou mixte.

Selon un autre mode, les particules sont recouvertes d'une couche de silice et d'hydroxyde ou oxyhydroxyde d'aluminium dans des teneurs en poids de 30 % de SiO2 et 15 % d'Al2O3 par rapport au dioxyde de titane.

Selon un mode plus particulier, les particules sont recouvertes d'une couche de silice et d'hydroxyde ou oxyhydroxyde d'aluminium dans des teneurs en poids de 15 % de SiO2 et 5 % d'Al2O3 par rapport au dioxyde de titane.

Selon la seconde de ces variantes, les particules d'oxyde de titane sont recouvertes au moins partiellement d'une première couche d'au moins un composé du cérium et/ou du fer, et d'une deuxième couche d'au moins un oxyde, hydroxyde ou oxyhydroxyde métallique ou de silicium. On pourra se référer pour cette seconde variante à la demande de brevet WO-A-98/01392 dont l'enseignement concernant la description du produit et son mode de préparation est incorporé ici.

Les composés présents dans la première couche précitée sont des précurseurs de l'oxyde de cérium ou de fer, c'est-à-dire qu'ils sont thermiquement décomposables en oxyde de cérium ou fer. Il peut s'agir de sels de cérium ou de fer.

Les particules recouvertes d'un composé du cérium sont préférées. Le rapport en poids du ou des composés du cérium sur le dioxyde de titane est de préférence d'au plus 6 % en poids, exprimée en CeO2. Ce rapport peut être optimisé en fonction de la taille des particules. Ainsi, il a été observé que pour des particules de diamètre 25 nm, la teneur optimale en cérium était de 5,5 % en poids, exprimée en CeO2, par rapport au dioxyde de titane; de même pour des particules de diamètre 45 nm, cette teneur est de 4,5 %; pour des particules de diamètre 60 nm, cette teneur est de 3 %, et pour des particules de diamètre 80 nm, cette teneur est de 2 %.

Les particules de cette deuxième variante sont également recouvertes au moins partiellement d'une deuxième couche à base d'au moins un oxyde, hydroxyde ou oxyhydroxyde métallique. L'oxyde est, en général, SiO2, quant à l'hydroxyde ou oxyhydroxyde métallique, il peut être en particulier choisi parmi les hydroxydes ou oxyhydroxydes de l'aluminium, de zinc, de titane ou d'étain sous forme simple ou mixte (au sens défini plus haut).

En général, le rapport en poids du ou des oxydes, hydroxydes ou oxyhydroxydes métalliques sur le dioxyde de titane est d'au plus 60 % en poids. Ce rapport est fonction de l'application à laquelle les particules sont destinées. De préférence, lorsque les particules sont utilisées en application cosmétique, ce rapport est d'au plus 25 %, encore plus préférentiellement d'au plus 20 %.

Ces quantités de composés, oxydes, hydroxydes ou oxyhydroxydes métalliques sont mesurées sur les particules en dispersion par fluorescence X.

Selon un mode de réalisation particulier, les particules sont recouvertes au moins partiellement d'une première couche d'un composé du cérium et d'une deuxième couche à base de silice et/ou d'un hydroxyde ou oxyhydroxyde d'aluminium sous forme simple ou mixte. Les teneurs en poids peuvent être dans ce cas de 15 % de SiO2 et 5 % d'Al2O3 par rapport au dioxyde de titane. La deuxième couche peut aussi être à base uniquement de silice dans une teneur en poids de 30 % de SiO2.

La phase liquide organique du sol de l'invention peut être à base d'un liquide organique ou d'un mélange de liquides organiques de nature très variée.

Le solvant ou liquide organique peut être un hydrocarbure aliphatique, cycloaliphatique inerte, ou leur mélange, tel que par exemple des essences minérales ou de pétrole pouvant contenir également des composants aromatiques. On peut citer à titre indicatif l'hexane, l'heptane, l'octane, le nonane, le décane, le cyclohexane, le cyclopentane, le cycloheptane et les naphtènes liquides. Les solvants aromatiques tels que le benzène, le toluène, l'éthylbenzène et les xylènes conviennent également ainsi que les coupes pétrolière du type ISOPAR ou SOLVESSO (marques déposées par la société EXXON), notamment le SOLVESSO 100 qui contient essentiellement un mélange de méthyléthyl et triméthylbenzène, et le SOLVESSO 150 qui renferme un mélange d'alcoyl benzènes en particulier de diméthyléthybenzène et de tétraméthylbenzène.

On peut mettre en oeuvre également des hydrocarbures chlorés tels que le chloro ou dichlorobenzène, le chlorotoluène, aussi bien que des éthers aliphatiques et cycloaliphatiques tels que l'éther de diisopropyle, l'éther de dibutyle et les cétones aliphatiques et cycloaliphatiques telles que la méthylisobutylcétone, la diisobutylcétone, l'oxyde de mésityle.

On peut aussi utiliser les cétones, comme l'acétone, les aldéhydes, les solvants azotés comme l'acétonitrile, les alcools, les acides et les phénols.

Les esters peuvent aussi être envisagés. On peut citer comme esters susceptibles d'être utilisés notamment ceux issus de la réaction d'acides avec des alcools en C1 à C8 et notamment les palmitates d'alcool secondaire tel l'isopropanol. Les acides dont sont issus ces esters peuvent être des acides carboxyliques aliphatiques, des acides sulfoniques aliphatiques, des acides phosphoniques aliphatiques, des acides alcoylarylsulfoniques, et des acides alcoylarylphosphoniques possédant environ de 10 à environ 40 atomes de carbone, qu'ils soient naturels ou synthétiques. A titre d'exemple, on peut citer les acides gras de tallol, d'huile de coco, de soja, de suif, d'huile de lin, l'acide oléique, l'acide linoléique, l'acide stéarique et ses isomères, l'acide pélargonique, l'acide caprique, l'acide laurique, l'acide myristique, l'acide dodécylbenzènesulfonique, l'acide éthyl-2 hexanoïque, l'acide naphténique, l'acide hexoïque, l'acide toluène-sulfonique, l'acide toluène-phosphonique, l'acide lauryl-sulfonique, l'acide lauryl-phosphonique, l'acide palmityl-sulfonique, et l'acide palmityl-phosphonique.

Selon une caractéristique particulièrement intéressante du sol de l'invention, la phase liquide organique est à base d'un solvant polaire ou d'un mélange de solvants polaires. Par solvant polaire on entend ceux présentant une constante diélectrique $\varepsilon_r$ supérieure à 5, telle que définie notamment dans l'ouvrage "Solvents and Solvent Effects in Organic Chemistry", C. Reichardt, VCH, 1988. Ce solvant polaire peut être choisi parmi les solvants halogénés comme le dichlorométhane; les esters du type acétate d'éthyle, palmitate d'isopropyle, méthoxy-propyl acétate; les alcools comme l'éthanol, le butanol ou l'isopropanol; les polyols tels que le propane diol, le butane diol ou le diéthylène glycol; les cétones comme la cyclohexanone ou la 1-méthylpyrrolidin-2 one.

Selon une caractéristique importante de l'invention, le sol comprend en outre un composé amphiphile. Sans vouloir être lié par une explication, on peut penser que ce composé amphiphile est adsorbé sur ou en interaction électrostatique avec les particules d'oxyde de titane ou encore complexé avec celles-ci.

Ce composé est choisi parmi les alkyl éthers phosphates polyoxyéthylénés. On entend ici les phosphates d'alcoyle polyoxyéthylénés de formule :

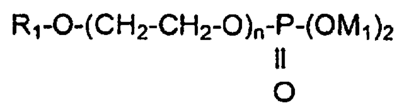

ou encore les phosphates de dialcoyle polyoxyéthylénés de formule :

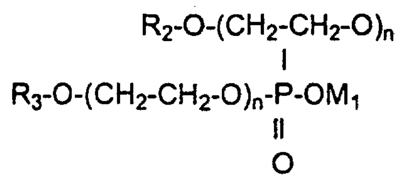

dans laquelle R1, R2, R3, identiques ou différents représentent un radical alkyl linéaire ou ramifié, notamment de 2 à 20 atomes de carbone; un radical phényle; un radical alkylaryl, plus particulièrement un radical alkylphényl, avec notamment une chaîne alkyle de 8 à 12 atomes de carbone; un radical arylalkyle, plus particulièrement un radical phénylaryl; n le nombre d'oxyde d'éthylène pouvant varier de 2 à 12 par exemple; $M_1$ représente un atome d'hydrogène, de sodium ou de potassium. Le radical $R_3$ peut être notamment un radical hexyle, octyle, décyle, dodécyle, oléyle, nonylphényle.

On peut citer comme exemple de ce type de composés amphiphiles ceux commercialisés sous les marques Lubrophos® et Rhodafac® et notamment les produits ci-dessous :

- les poly-oxy-ethylène alkyl (C8-C10) ethers phosphates Rhodafac® RA 600
- les poly-oxyethylène tri-decyl ether phosphate Rhodafac® RS 710 ou RS 410
- le poly-oxy-ethylène oleocétyl ether phosphate Rhodafac® PA 35
- le poly-oxy-ethylène nonylphenyl ether phosphate Rhodafac® PA 17
- le poly-oxy-ethylène nonyl(ramifié) ether phosphate Rhodafac® RE 610

Le choix du composé amphiphile se fait en fonction de la nature de la phase liquide organique. Plus précisément, ce choix se fait en adaptant l'équilibre hydrophile/lipophile du composé amphiphile au caractère hydrophile/lipophile de la phase organique. En d'autres termes, plus le solvant entrant dans la constitution de la phase organique est polaire, plus le composé amphiphile sera hydrophile.

La proportion de composé amphiphile par rapport à l'oxyde de titane est généralement comprise entre 2 et 10 molécules par $nm^2$ de surface d'oxyde de titane, en supposant une surface par tête complexant du cation titane comprise entre 10 et 80Œ$^2$.

Les sols selon l'invention présentent une concentration en composé de titane qui peut aller jusqu'à 40% exprimée en poids de $TiO_2$ par rapport au poids total de la dispersion.

Les sols organiques ainsi élaborés présentent une excellente stabilité. On n'observe pas de décantation au bout de plusieurs mois.

La présente invention concerne aussi un composé solide qui est caractérisé en ce qu'il comprend un mélange de particules d'oxyde de titane et d'au moins un composé amphiphile choisi parmi ceux qui ont été décrits plus haut.

Ce composé solide se présente soit sous forme d'une pâte soit sous forme d'une poudre. L'oxyde de titane se présente dans ce composé solide sous forme de cristallites élémentaires agrégés, la taille moyenne des agrégats pouvant aller de 20 à 100nm. Le composé solide présente la propriété d'être redispersible, c'est à dire de pouvoir donner un sol selon l'invention et tel que décrit plus haut lorsqu'il est mis en suspension dans une phase liquide organique.

Les particules d'oxyde de titane du composé solide selon l'invention peuvent comporter un revêtement au moins partiel sous forme d'une couche d'au moins un oxyde, hydroxyde ou oxyhydroxyde métallique ou de silicium ou encore sous forme d'une première couche d'au moins un composé du cérium et/ou du fer, et d'une deuxième couche d'au moins un oxyde, hydroxyde ou oxyhydroxyde métallique ou de silicium.

Ce qui a été décrit plus haut par ailleurs pour les particules du sol s'applique aussi ici pour le composé solide.

Les procédés de préparation du composé solide et du sol de l'invention vont maintenant être décrits.

On utilise comme produit de départ tout oxyde de titane susceptible de pouvoir donner un sol quand il est mis en dispersion dans une phase liquide et notamment tout oxyde de titane susceptible de se présenter sous la forme qui a été décrite plus haut au sujet du composé solide.

On donne ci-dessous la description et le procédé de préparation de particules d'oxyde de titane qui conviennent particulièrement bien comme produit de départ pour la préparation du sol et du composé solide selon l'invention.

Les particules de départ sont à base de dioxyde de titane de structure cristalline majoritairement anatase, ainsi que défini précédemment.

Ces particules de dioxyde de titane anatase de départ peuvent présenter une taille d'au plus 100 nm, de préférence d'au moins 15 nm, encore plus préférentiellement compris entre 20 et 70 nm. Ce diamètre est mesuré par microscopie électronique par transmission (MET). Leur surface spécifique BET est généralement d'au moins 200 $m^2/g$, de préférence d'au moins 250 $m^2/g$. Cette surface spécifique BET est mesurée de la même manière que définie précédemment.

Les particules de départ présentent également une densité de l'ordre de 2,5. Par "de l'ordre", on entend que la densité est de 2,5 ± 0,2. Cette densité est donnée par la formule suivante :

$$\text{densité} = \frac{1}{(1/\rho) + Vi}$$

dans laquelle :

- $\rho$ est la densité de l'anatase, soit 3,8,
- Vi est le volume apporté par les pores intra particules, il est mesuré par la méthode BJH. On entend par volume mesuré par la méthode BJH, la volume mesuré à partir de la méthode BARRETT-JOYNER-HELENDA décrite dans l'article de l'ouvrage Techniques de l'Ingénieur, et intitulé "Texture des solides poreux ou divisés", p.3645-1 à 3645-13.

Pour mesurer le volume apporté par les pores intra particules des particules selon l'invention, lorsqu'elles se présentent sous forme de dispersion, il est essentiel de suivre le protocole de mesure qui consiste à éliminer la phase liquide de la dispersion puis à sécher les particules sous vide à une température de 150°C pendant au moins 4 heures.

Des particules telles que décrites ci-dessus peuvent être obtenues par hydrolyse d'au moins un composé du titane A en présence d'au moins un composé B choisi parmi :

(i) les acides qui présentent :

- soit un groupement carboxyle et au moins deux groupements hydroxyles et/ou amines,
- soit au moins deux groupements carboxyles et au moins un groupement hydroxyle et/ou amine,

(ii) les acides phosphoriques organiques de formules suivantes :

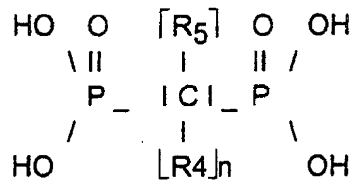

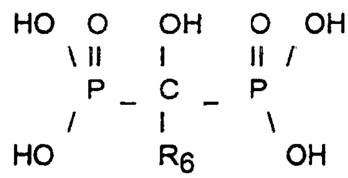

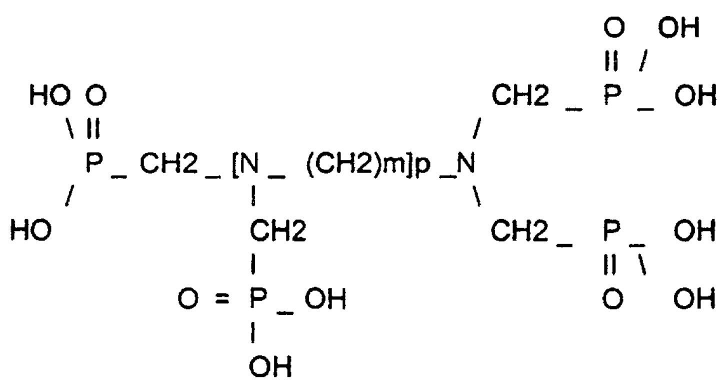

dans lesquelles, n et m sont des nombres entiers compris entre 1 et 6, p est un nombre entier compris entre 0 et 5, $R_4$, $R_5$, $R_6$ identiques ou différents représentant un groupement hydroxyle, amino, aralkyl, aryl, alkyl ou l'hydrogène,

(iii) les composés capables de libérer des ions sulfates en milieu acide,

(iv) les sels des acides décrits ci-dessus,

et en présence de germes de dioxyde de titane anatase.

La solution de départ, destinée à être hydrolysée, est de préférence totalement aqueuse ; éventuellement on peut ajouter un autre solvant, un alcool par exemple, à condition que le composé du titane A et le composé B utilisés soient alors substantiellement solubles dans ce mélange.

En ce qui concerne le composé du titane A, on utilise en général un composé choisi parmi les halogénures, les oxyhalogénures, les alcoxydes de titane, les sulfates et plus particulièrement les sulfates synthétiques.

On entend par sulfates synthétiques des solutions de sulfates de titanyle réalisées par échange d'ions à partir de solutions de chlorure de titane très pures ou par réaction d'acide sulfurique sur un alcoxyde de titane.

De préférence, on opère avec des composés du titane du type halogénure ou oxyhalogénure de titane. Les halogénures ou les oxyhalogénures de titane plus particulièrement utilisés dans la présente invention sont les fluorures, les chlorures, les bromures et les iodures (respectivement les oxyfluorures, les oxychlorures, les oxybromures et les oxyiodures) de titane.

Selon un mode particulièrement préféré, le composé du titane est l'oxychlorure de titane $TiOCl2$.

La quantité de composé de titane A présente dans la solution à hydrolyser n'est pas critique.

La solution initiale contient en outre au moins un composé B tel que défini précédemment. A titre d'exemples non limitatifs de composés B entrant dans le cadre de la présente invention, on peut citer notamment :

- les acides hydroxypolycarboxyliques, et plus particulièrement les acides hydroxydi- ou hydroxytricarboxyliques tels que l'acide citrique, l'acide maléique et l'acide tartrique.
- les acides (polyhydroxy)monocarboxyliques, comme par exemple l'acide glucoheptonique et l'acide gluconique,
- les acides poly(hydroxycarboxyliques), comme par exemple l'acide tartrique,
- les monoacides dicarboxyliques et leurs amides correspondantes, comme par exemple l'acide aspartique, l'asparagine et l'acide glutamique,
- les aminoacides monocarboxyliques, hydroxylés ou non, comme par exemple la lysine, la sérine et la thréonine,
- l'aminotriphosphonate de méthylène, l'éthylènediaminotétraphosphonate de méthylène, le triéthylènetétraaminohexaphosphonate de méthylène, le tétraéthylènepentaaminoheptaphosphonate de méthylène, le pentaéthylènehexaaminooctaphosphonate de méthylène,
- le diphosphonate de méthylène; de 1,1' éthylène; de 1,2 éthylène; de 1,1' propylène; de 1,3 propylène; de 1,6 hexaméthylène; le 2,4 dihydroxypentaméthylène - 2,4 diphosphonate; le 2,5 dihydroxyhexaméthylène - 2,5 disphosphonate ; le 2,3 dihydroxybutylène - 2,3 diphosphonate ; le 1 hydroxybenzyle - 1,1' diphosphonate ; le 1 aminoéthylène 1-1' diphosphonate ; l'hydroxyméthylène diphosphonate ; le 1 hydroxyéthylène 1,1' diphosphonate ; le 1 hydroxypropylène 1-1' diphosphonate ; le 1 hydroxybutylène 1-1' diphosphonate ; le 1 hydroxyhexaméthylène - 1,1' diphosphonate.

Comme déjà indiqué, il est également possible d'utiliser à titre de composé B tous les sels des acides précités. En particulier, ces sels sont soit des sels alcalins, et plus particulièrement des sels de sodium, soit des sels d'ammonium.

Ces composés peuvent être choisis aussi parmi l'acide sulfurique et les sulfates d'ammonium, de potassium notamment.

De préférence, les composés B tels que définis ci-dessus sont des composés hydrocarbonés de type aliphatique. Dans ce cas, la longueur de la chaîne principale hydrocarbonée n'excède pas de préférence 15 atomes de carbone, et plus préférentiellement 10 atomes de carbone. Le composé B préféré est l'acide citrique.

La quantité de composé B n'est pas critique. D'une manière générale, la concentration molaire du composé B par rapport à celle du composé du titane A est comprise entre 0,2 et 10 % et de préférence entre 1 et 5 %.

Enfin la solution de départ comprend des germes de dioxyde de titane. On donne ci-dessous les caractéristiques spécifiques péférentielles de ces germes et de leur mise en oeuvre.

Ainsi, les germes de dioxyde de titane utilisés dans la présente invention présentent tout d'abord une taille inférieure à 8 nm, mesurée par diffraction X. De préférence, on utilise des germes de dioxyde de titane présentant une taille comprise entre 3 et 5 nm.

Ensuite, le rapport pondéral du dioxyde de titane présent dans les germes sur le titane présent dans le milieu d'hydrolyse avant introduction des germes - c'est-à-dire apporté par le composé du titane A - et exprimé en $TiO2$ est compris entre 0,01 et 3 %. Ce rapport peut être préférentiellement compris entre 0,05 et 1,5 %. La réunion de ces deux conditions sur les germes (taille et rapport pondéral) associée au procédé tel que décrit précédemment permet de contrôler précisément la taille finale des particules de dioxyde de titane en associant à un taux de germes une taille de particule. On peut ainsi obtenir des particules dont le diamètre varie entre 20 et 100 nm.

On utilise des germes de dioxyde de titane sous forme anatase de manière à induire la précipitation du dioxyde de titane sous forme anatase. Généralement, du fait de leur petite taille, ces germes se présentent plutôt sous la forme d'anatase mal cristallisé. Les germes se présentent habituellement sous la forme d'une suspension aqueuse constituée de dioxyde de titane. Ils peuvent être généralement obtenus de manière connue par un procédé de neutralisation d'un sel de titane par une base.

L'étape suivante consiste à réaliser l'hydrolyse de la solution de départ par tout moyen connu de l'homme du métier et en général par chauffage. Dans ce dernier cas, l'hydrolyse peut de préférence être effectuée à une température supérieure ou égale à 70°C. On peut aussi travailler dans un premier temps à une température inférieure à la température d'ébullition du milieu puis maintenir le milieu d'hydrolyse en palier à la température d'ébullition.

Une fois l'hydrolyse réalisée, les particules de dioxyde de titane obtenues sont récupérées par séparation du solide précipité des eaux mères. Elles peuvent être redispersées dans un milieu liquide, de préférence acide ou basique, par exemple dans de l'eau, de manière à obtenir une dispersion de dioxyde de titane.

Selon une variante de procédé, après la récupération des particules obtenues à la suite de l'hydrolyse et avant leur remise en dispersion, on peut neutraliser les particules et/ou leur faire subir au moins un lavage. Les particules peuvent être récupérées par exemple par centrifugation de la solution issue de l'hydrolyse, elles sont ensuite neutralisées par une base, par exemple une solution d'ammoniaque ou de soude. On peut les laver en les redispersant dans une solution aqueuse puis les particules sont séparées de la phase aqueuse de lavage. Après éventuellement un ou plusieurs autres lavages du même type, les particules sont remises en dispersion dans un liquide, par exemple de l'eau, qui, de préférence, peut être acide ou basique.

En ce qui concerne les particules d'oxyde de titane sous forme rutile, celles-ci peuvent être obtenues par

hydrolyse d'un composé du titane choisi parmi les halogénures, les oxyhalogénures et les alcoxydes de titane.

Il est possible d'utiliser les particules de titane sous forme solide par simple évaporation ou par séchage à une température d'au plus 120°C de la dispersion précitée, c'est à dire celle obtenue après hydrolyse, séparation du milieu d'hydrolyse et remise dans une phase liquide. Cette température est de préférence comprise entre 30 et 80°C.

En ce qui concerne la préparation des particules d'oxyde de titane selon les deux variantes qui ont été décrites plus haut, c'est à dire celles dans lesquelles les particules comprennent un revêtement, on procède selon l'enseignement des demandes de brevets EP-A-880564 et WO-A-98/01392 mentionnées plus haut. On peut rappeler ici, dans le cas de la première variante, que le procédé consiste à précipiter au moins un oxyde, hydroxyde ou oxyhydroxyde métallique à la surface de particules de dioxyde de titane. Cette précipitation peut être réalisée par introduction, dans une dispersion de particules de dioxyde de titane de précurseurs des oxydes, hydroxydes ou oxyhydroxydes métalliques en général sous forme de solutions aqueuses de sels, puis modification du pH pour obtenir la précipitation de ces oxydes, hydroxydes ou oxyhydroxydes sur les particules de dioxyde de titane.

Dans le cas de la deuxième variante, le procédé consiste à précipiter au moins un composé du cérium et/ou du fer à la surface de particules de dioxyde de titane puis à précipiter au moins un oxyde, hydroxyde ou oxyhydroxyde métallique à la surface de particules obtenues.

Ces précipitations peuvent être réalisées, là aussi, par introduction, dans une dispersion de particules de dioxyde de titane de précurseurs des composés du cérium et/ou du fer, oxydes, hydroxydes ou oxyhydroxydes métalliques en général sous forme de solutions aqueuses de sels, puis, par modification du pH pour obtenir la précipitation de ces composés, oxydes, hydroxydes ou oxyhydroxydes sur les particules de dioxyde de titane.

En général et pour les deux variantes, on effectue cette précipitation à une température d'au moins 50°C.

Le procédé de préparation du sol de l'invention peut être mis en oeuvre selon une première variante. Dans cette variante, on mélange le composé amphiphile précité et la phase liquide organique, puis on disperse les particules d'oxyde de titane dans le mélange obtenu. Il est à noter que l'on peut soit introduire les particules solides dans le mélange composé amphiphile/phase organique soit verser ce mélange sur les particules d'oxyde de titane. Une fois les particules, le composé amphiphile et la phase organique mis en présence, on agite jusqu'à l'obtention d'une dispersion colloïdale stable.

Il existe une seconde variante du procédé. Dans ce cas, on forme un mélange d'oxyde de titane et d'au moins un composé amphiphile précité. Ce mélange peut être fait en utilisant tout moyen mécanique connu tel que le malaxage pour obtenir une pâte homogène. On obtient ainsi un composé solide tel que défini plus haut. Dans un deuxième temps, on disperse dans la phase liquide organique ledit mélange.

Une troisième variante va maintenant être décrite qui convient plus particulièrement à la préparation d'un sol en phase organique polaire.

Cette variante, pour la préparation d'un sol selon l'invention dans une phase liquide organique (a) comprend une première étape dans laquelle on forme une dispersion comprenant des particules d'oxyde de titane et au moins un composé amphiphile du type précité dans une phase liquide organique (b) à base d'un solvant de polarité plus faible que celle du solvant de la phase liquide organique (a). On peut observer lors de la formation de cette dispersion une démixtion due à l'eau éventuellement présente dans l'oxyde de titane hydraté de départ. Dans ce cas, on sépare l'eau démixée du reste de la dispersion.

Dans une seconde étape, on sépare la phase solide de la dispersion de la phase liquide (b) de celle-ci. Cette séparation peut se faire par toute technique convenable. On peut ainsi effectuer la séparation par floculation par un tiers solvant ou encore par distillation ou évaporation. On recueille à la suite de cette séparation une phase solide qui peut être séchée et qui se présente, suivant le niveau de séchage atteint, soit sous forme de poudre soit sous forme de pâte et qui constitue un composé solide selon l'invention. Dans une dernière étape, on redisperse la phase ou le composé solide ainsi obtenu dans la phase organique (a) pour obtenir ainsi le sol recherché.

Les dispersions des particules d'oxyde de titane dans la phase organique peuvent être soumises à un traitement d'ultrafiltration pour améliorer leur stabilité si nécessaire.

On notera enfin que les sols obtenus peuvent subir un post-traitement de déshydratation par passage sur un agent de dessiccation solide par exemple.

Les sols de l'invention peuvent être utilisés dans toutes les applications où le titane est employé pour ses propriétés photocatalytiques. Dans ce cas, les particules d'oxyde de titane ne comportent pas de revêtement du type décrit plus haut.

Les sols à base de particules d'oxyde de titane de l'invention et notamment ceux comportant des particules avec revêtement de ce type peuvent être utilisés en tant qu'agent anti-UV dans la préparation de formulations pour cosmétiques, vernis, peintures et dans les plastiques.

Des exemples non limitatifs vont maintenant être donnés.

EXEMPLE 1-A

Cet exemple et le suivant concerne la préparation d'une dispersion colloïdale en milieu Isopar. On prépare tout d'abord les particules d'oxyde de titane de la manière suivante.

On ajoute successivement à 394,7 g d'une solution d'oxychlorure de titane à 1,9 mol/kg :

- 42,02 g d'acide chlorhydrique à 36 %,
- 4,73 g d'acide citrique,
- 547,1 g d'eau épurée,
- 73,84 g d'une suspension contenant 1,06% en poids de germes d'anatase.

Le mélange est porté à ébullition et y est maintenu pendant 3 heures. La solution est laissée à décantée et on soutire le surnageant par siphonnage. On remet en dispersion par de l'eau déminéralisée par un volume d'eau de manière à obtenir un extrait sec de 20% en poids. On obtient ainsi un sol parfaitement stable. La taille des colloïdes est de 22 nm.

Ensuite, on dissout préalablement 11,2g d'ester phosphate Rhodafac RS 410, commercialisé par Rhodia, dans 70g d'Isopar L à température ambiante et sous agitation. On introduit dans ce mélange de façon progressive 10g de poudre de TiO2. Cette poudre de TiO2 a été obtenue par séchage à 50°C de la dispersion colloïdale d'oxyde de TiO2 précédemment décrite et contient 77 % de TiO2. On complète par de l'Isopar jusqu'à une masse totale (composé amphiphile + poudre de $TiO_2$ + Isopar) égale à 100g. On laisse sous agitation jusqu'à obtention d'une dispersion colloïdale stable. On observe une taille des particules sensiblement identique à la taille des particules présentes dans la dispersion colloïdale aqueuse initiale.

EXEMPLE 1-B

On introduit 26g de poudre de TiO2, obtenue par séchage à 50°C de la dispersion colloïdale décrite à l'exemple 1-a. On ajoute 29,9g d'ester phosphate RS 410 et on complète par du solvant CH2Cl2 jusqu'à 336g. On laisse sous agitation une nuit jusqu'à obtention d'une dispersion.

Une aliquote de 168g de dispersion est mise à évaporer au rotavapor à 30°C sous vide obtenue par une trompe à eau. La poudre ainsi recueillie est mise à redisperser dans un volume équivalent d'Isopar. On obtient une dispersion dans l'Isopar, stable au cours du temps.

EXEMPLE 2-A

Cet exemple et le suivant concerne la préparation d'une dispersion colloïdale en milieu xylène.

On dissout préalablement 15,6g d'ester phosphate Rhodafac RS 710, commercialisé par Rhodia, dans 60g de xylène, à température ambiante et sous agitation. On introduit dans ce mélange, de manière progressive, 10g de poudre de TiO2, obtenue par séchage à 50°C de la dispersion colloïdale d'oxyde de TiO2 précédemment décrite, et on complète à température ambiante par du xylène jusqu'à une masse totale de 100g . On laisse sous agitation jusqu'à obtention d'une dispersion colloïdale stable. On observe une taille des particules sensiblement identique à la taille des particules présentes dans la dispersion colloïdale aqueuse initiale.

EXEMPLE 2-B

On introduit 26g de poudre de TiO2, obtenue par séchage à 50°C de la dispersion colloïdale décrite à l'exemple 1-a. On ajoute 29,9g d'ester phosphate RS 410 et on complète par du solvant CH2Cl2 jusqu'à 336g. On laisse sous agitation une nuit jusqu'à obtention d'une dispersion.

Une aliquote de 168g de dispersion est mise à évaporer au rotavapor à 30°C sous vide obtenue par une trompe à eau. La pâte ainsi recueillie est mise à redisperser dans un volume équivalent de xylène. On obtient une dispersion dans le xylène, stable au cours du temps.

EXEMPLE 3

Cet exemple concerne une dispersion colloïdale en milieu acétate d'éthyle.

On dissout préalablement 1,93g d'ester phosphate Rhodafac® RS 710, commercialisé par Rhodia, dans 15g de xylène, à température ambiante et sous agitation. On ajoute de manière progressive 2g de poudre de TiO2, obtenue par séchage à 50°C de la dispersion colloïdale d'oxyde de TiO2 précédemment décrite, et on complète à température ambiante à 20g par du xylène. On laisse sous agitation jusqu'à obtention d'une dispersion colloïdale stable.

5g de la dispersion colloïdale ainsi obtenue sont mis à évaporer à température ambiante sous hotte ventilée. La pâte ainsi obtenue est mise à redisperser dans 4,11g d'acétate d'éthyle. Après agitation, on obtient une dispersion colloïdale stable au cours du temps. On observe une taille des particules de 24 nm, sensiblement identique à la taille des particules présentes dans la dispersion colloïdale aqueuse initiale.

EXEMPLE 4

Cet exemple concerne une dispersion colloïdale en milieu butanol.

On dissout préalablement 0,77g d'ester phosphate Rhodafac® RS 710, commercialisé par Rhodia, dans 15g de xylène, à température ambiante et sous agitation. On introduit 2g de poudre de TiO2, obtenue par séchage à 50°C de la dispersion colloïdale d'oxyde de TiO2 précédemment décrite, et on complète à température ambiante par du xylène jusqu'à obtenir une masse totale de 20g. On laisse sous agitation jusqu'à obtention d'une dispersion colloïdale stable.

5g de la dispersion colloïdale ainsi obtenue sont mis à évaporer à température ambiante sous hotte ventilée. La pâte ainsi obtenue est mise à redisperser dans 4,35g de butanol. Après agitation, on obtient une dispersion colloïdale stable au cours du temps. On observe une taille des particules de 28 nm.

EXEMPLE 5

Cet exemple concerne un dispersion colloïdale en milieu éthanol.

On dissout préalablement 1,93g d'ester phosphate RS 710, commercialisé par Rhodia, dans 15g de xylène, à température ambiante et sous agitation. On ajoute de manière progressive 2g de poudre de TiO2, obtenue par séchage à 50°C de la dispersion colloïdale d'oxyde de TiO2 précédemment décrite, et on complète à température ambiante à 20g par du xylène. On laisse sous agitation jusqu'à obtention d'une dispersion colloïdale stable.

5g de la dispersion colloïdale ainsi obtenue sont mis à évaporer à température ambiante sous hotte ventilée. La pâte ainsi obtenue est mise à redisperser dans 4,11g d'éthanol absolu. Après agitation, on obtient une dispersion colloïdale stable au cours du temps. On observe une taille des particules de 23 nm.

EXEMPLE 6

Cet exemple concerne une dispersion colloïdale de TiO2 revêtu par Al203 et SiO2 dans l'Isopar.

On utilise une dispersion colloïdale de nanoparticules de TiO2 traitée en surface selon le mode opératoire du type décrit dans l'exemple 2 de la demande de brevet EP-A-880564, les particules d'oxyde de titane de départ ayant été obtenues selon l'exemple 1 de cette même demande et par ajout à la solution d'oxychlorure de titane de 17,04g d'une suspension contenant 1,06% en poids de germes d'anatase ayant une taille comprise entre 5 et 6 nm. La dispersion colloïdale possède un extrait sec d'environ 35%. des colloïdes de taille de 60 nm. La composition du revêtement de surface est de 15% SiO2-5% Al2O3, le pH de la dispersion est de 8,7.

Cette dispersion colloïdale est mise à évaporer à 50°C en étuve ventilée, une nuit.

On obtient une poudre à 85,5% d'oxyde.

On prépare une solution à 12,5% en poids de Rhodafac RS 410 dans l'isopar.

A 2g de la poudre séchée obtenue comme décrit précédemment, on ajoute 18g de la solution d'Isopar à 12,5%. On obtient alors une dispersion colloïdale stable au cours du temps.

EXEMPLE 7

Cet exemple concerne une dispersion colloïdale de TiO2 revêtu par Al203 et SiO2 dans le palmitate d'isopropyle.

A 2,34g de poudre de TiO2 traitée en surface comme décrite précédemment à l'exemple 6, on ajoute 0,44g de Rhodafac RS 410 et 18g de palmitate d'isopropyle. Après mise sous agitation à température ambiante, on obtient une dispersion colloïdale, stable au cours du temps.

EXEMPLE 8

Cet exemple concerne une dispersion colloïdale de TiO2 revêtu par Al203 et SiO2 dans le palmitate d'isopropyle et à forte concentration en oxyde de titane.

On solubilise 11,2g de Rhodafac RS 410 dans 80g de palmitate d'isopropyle. On ajoute alors lentement et sous agitation 60g de poudre de TiO2/Al2O3-SiO2 élaborée comme décrit précédemment à l'exemple 6. Après agitation 3 jours à température ambiante, on obtient une dispersion. Après dilution dans le palmitate d'isopropyle, on détermine

une taille de l'ordre de 63nm, sensiblement identique à la taille des nanoparticules de la dispersion initiale.

EXEMPLE 9

Cet exemple concerne une dispersion colloïdale de TiO2 revêtu de deux couches, l'une à base de CeO2 l'autre à base de Al203 et SiO2 dans le palmitate d'isopropyle.

La dispersion colloïdale de nanoparticules de TiO2 traitée en surface selon le mode opératoire décrit dans l'exemple 2 de WO-A-98/01392, les particules d'oxyde de titane de départ ayant été obtenues selon l'exemple 1 de cette même demande et par ajout à la solution d'oxychlorure de titane de 11,36g d'une suspension contenant 1,06% en poids de germes d'anatase présentant une taille comprise entre 5 et 6nm. Cette dispersion possède un extrait sec d'environ 23%, des colloïdes de taille de 45nm. La composition du traitement de surface est de 15% SiO2-5% Al2O3-5% CeO2 , le pH de la dispersion est pH 5,5.

Cette dispersion colloïdale est mise à évaporer à 50°C en étuve ventilée, une nuit.

On obtient une poudre à 85,3% en oxyde.

On solubilise 11,2g de Rhodafac RS 410 dans 80g de palmitate d'isopropyle. On ajoute alors lentement et sous agitation 60g de poudre de TiO2 Al2O3-SiO2 -CeO2 élaborée comme décrit précédemment. Après agitation 3 jours à température ambiante, on obtient une dispersion stable au cours du temps. Par analyse granulométrique par dilution dans le palmitate d'isopropyle, on détermine une taille de l'ordre de 72 nm, sensiblement identique à la taille des nanoparticules de la dispersion initiale.

## Revendications

1. Sol organique, **caractérisé en ce qu'**il comprend :

    - des particules d'oxyde de titane;
    - une phase liquide organique;
    - au moins un composé amphiphile choisi parmi les alkyl éthers phosphates polyoxyéthylénés.

2. Sol selon la revendication 1, **caractérisé en ce que** les particules d'oxyde de titane sont recouvertes au moins partiellement d'une couche d'au moins un oxyde, hydroxyde ou oxyhydroxyde métallique ou de silicium.

3. Sol selon la revendication 1, **caractérisé en ce que** les particules d'oxyde de titane sont recouvertes au moins partiellement :

    - d'une première couche d'au moins un composé du cérium et/ou du fer, et
    - d'une deuxième couche d'au moins un oxyde, hydroxyde ou oxyhydroxyde métallique

    ou de silicium.

4. Sol selon la revendication 2 ou 3, **caractérisé en ce que** les particules d'oxyde de titane présentent une surface spécifique BET d'au moins 70 $m^2/g$.

5. Sol selon l'une des revendications 2 à 4, **caractérisé en ce que** le rapport en poids du ou des oxydes, hydroxydes ou oxyhydroxydes métalliques ou de silicium sur le dioxyde de titane est d'au plus 60 % en poids.

6. Sol selon l'une des revendications 3 à 5, **caractérisé en ce que** la première couche précitée est à base d'au moins un composé du cérium dans une teneur telle que le rapport en poids du composé du cérium, exprimé en Ce02, sur le dioxyde de titane est d'au plus 6 % en poids.

7. Sol selon l'une des revendications 2 à 6, **caractérisé en ce** la couche précitée ou la deuxième couche précitée est à base de silice et/ou d'oxyde, d'hydroxyde ou d'oxyhydroxyde d'aluminium.

8. Sol selon l'une des revendications précédentes, **caractérisé en ce que** la phase liquide organique est à base d'un solvant polaire.

9. Sol selon l'une des revendications précédentes, **caractérisé en ce que** le composé amphiphile est choisi parmi les alkyl ou alkylaryl éthers phosphates polyoxyéthylénés.

**10.** Sol selon l'une des revendications précédentes, **caractérisé en ce que** le solvant polaire est choisi parmi les solvants halogénés, les esters, les alcools.

**11.** Composé solide, **caractérisé en ce qu'**il comprend un mélange de particules d'oxyde de titane et d'au moins un composé amphiphile choisi parmi les alkyl éthers phosphates polyoxyéthylénés.

**12.** Composé solide selon la revendication 11, **caractérisé en ce que** les particules d'oxyde de titane sont recouvertes au moins partiellement d'une couche d'au moins un oxyde, hydroxyde ou oxyhydroxyde métallique ou de silicium.

**13.** Composé solide selon la revendication 11, **caractérisé en ce que** les particules d'oxyde de titane sont recouvertes au moins partiellement :

- d'une première couche d'au moins un composé du cérium et/ou du fer, et
- d'une deuxième couche d'au moins un oxyde, hydroxyde ou oxyhydroxyde métallique

ou de silicium.

**14.** Procédé de préparation d'un sol selon l'une des revendications 1 à 10, **caractérisé en ce qu'**on mélange le composé amphiphile précité et la phase liquide organique, puis on disperse les particules d'oxyde de titane, éventuellement recouvertes de la ou des deux couches précitées, dans le mélange obtenu.

**15.** Procédé de préparation d'un sol selon l'une des revendications 1 à 10, **caractérisé en ce qu'**on forme un mélange de particules d'oxyde de titane, éventuellement recouvertes de la ou des deux couches précitées, et d'au moins un composé amphiphile précité puis on disperse dans la phase liquide organique ledit mélange.

**16.** Procédé de préparation d'un sol selon l'une des revendications 1 à 10 comprenant une phase liquide organique (a), notamment d'un sol dans une phase organique (a) à base d'un solvant polaire, **caractérisé en ce qu'**on forme. une dispersion comprenant des particules d'oxyde de titane, éventuellement recouvertes de la ou des deux couches précitées, et au moins un composé amphiphile précité dans une phase liquide organique (b) à base d'un solvant de polarité plus faible que celle du solvant de la phase liquide organique (a); on sépare la phase solide de la phase liquide (b); on disperse la phase solide ainsi obtenue dans la phase organique (a).

**17.** Procédé de préparation selon l'une des revendications 14 à 16, **caractérisé en ce qu'**on utilise comme produit de départ des particules de dioxyde de titane qui ont été obtenues par hydrolyse d'au moins un composé du titane A en présence d'au moins un composé B choisi parmi :

(i) les acides qui présentent :

- soit un groupement carboxyle et au moins deux groupements hydroxyles et/ou amines,
- soit au moins deux groupements carboxyles et au moins un groupement hydroxyle et/ou amine,

(ii) les acides phosphoriques organiques de formules suivantes :

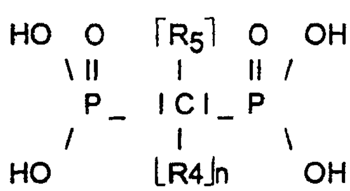

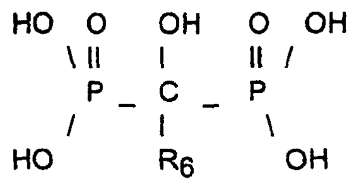

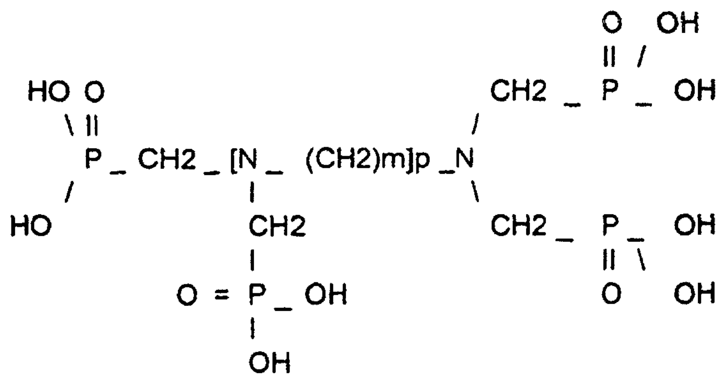

dans lesquelles, n et m sont des nombres entiers compris entre 1 et 6, p est un nombre entier compris entre 0 et 5, $R_4$, $R_5$, $R_6$ identiques ou différents représentant un représentant un groupement hydroxyle, amino, aralkyl, aryl, alkyl

ou l'hydrogène,

(iii) les composés capables de libérer des ions sulfates en milieu acide,

(iv) les sels des acides décrits ci-dessus,

et en présence de germes de dioxyde de titane anatase;
puis séparation du précipité formé du milieu d'hydrolyse.

**18.** Procédé selon la revendication 17, **caractérisé en ce qu'**on utilise comme produit de départ des particules de dioxyde de titane qui ont été obtenues par le procédé d'hydrolyse précité et dans lequel les germes de dioxyde de titane anatase ont une taille d'au plus 8 nm et sont présent dans un rapport pondéral exprimé en TiO2 présent dans les germes/titane présent avant introduction des germes dans le milieu d'hydrolyse, exprimé en TiO2 compris entre 0.01 % et 3 %.

**19.** Procédé selon la revendication 17 ou 18, **caractérisé en ce qu'**on utilise comme produit de départ des particules de dioxyde de titane qui ont été obtenues par le procédé d'hydrolyse précité et dans lequel le composé du titane A est l'oxychlorure de titane.

**20.** Procédé selon l'une des revendications 17 à 19, **caractérisé en ce qu'**on utilise comme produit de départ des particules de dioxyde de titane qui ont été obtenues par le procédé d'hydrolyse précité et dans lequel le composé B est l'acide citrique.

**21.** Procédé selon l'une des revendications 17 à 20, **caractérisé en ce qu'**on utilise comme produit de départ des particules de dioxyde de titane qui ont été obtenues par un procédé comprenant l'hydrolyse précitée et dans lequel le précipité formé est séparé du milieu d'hydrolyse puis est remis en dispersion dans l'eau ce par quoi on obtient une dispersion d'oxyde de titane dans l'eau et où on sèche ladite dispersion à une température d'au plus 120°C.

**22.** Procédé selon l'une des revendications 14 à 21, **caractérisé en ce qu'**on soumet le sol à un traitement d'ultrafiltration.

**23.** Utilisation d'un sol selon l'une des revendications 1 à 10 pour la préparation de formulations pour cosmétiques, vernis, peintures et dans les plastiques.

## Patentansprüche

1. Organisches Sol, **dadurch gekennzeichnet, daß** es enthält:

   - Titanoxidteilchen;
   - eine flüssige organische Phase;
   - mindestens eine amphiphile Verbindung, ausgewählt aus polyoxyethylenierten Alkyletherphosphaten.

2. Sol nach Anspruch 1, **dadurch gekennzeichnet, daß** die Titanoxidteilchen zumindest teilweise mit einer Schicht mindestens eines Metall- oder Siliciumoxids, -hydroxids oder -oxyhydroxids bedeckt sind.

3. Sol nach Anspruch 1, **dadurch gekennzeichnet, daß** die Titanoxidteilchen zumindest teilweise bedeckt sind:

   - mit einer ersten Schicht mindestens einer Cer- und/oder Eisenverbindung und
   - mit mindestens einer zweiten Schicht mindestens eines Metall- oder Siliciumoxids, -hydroxids oder -oxyhydroxids.

4. Sol nach Anspruch 2 oder 3, **dadurch gekennzeichnet, daß** die Titanoxidteilchen eine spezifische BET-Oberfläche von mindestens 70 $m^2/g$ aufweisen.

5. Sol nach einem der Ansprüche 2 bis 4, **dadurch gekennzeichnet, daß** das Gewichtsverhältnis des oder der Metall- oder Siliciumoxide, -hydroxide oder -oxyhydroxide zum Titandioxid höchstens 60 Gew.-% beträgt.

6. Sol nach einem der Ansprüche 3 bis 5, **dadurch gekennzeichnet, daß** die vorgenannte erste Schicht auf Basis mindestens einer Cerverbindung mit einem solchen Gehalt ausgebildet ist, daß das Gewichtsverhältnis der Cerverbindung, berechnet als $CeO_2$, in bezug auf das Titandioxid höchstens 6 Gew.-% beträgt.

7. Sol nach einem der Ansprüche 2 bis 6, **dadurch gekennzeichnet, daß** die vorgenannte Schicht oder die vorgenannte zweite Schicht auf Basis eines Siliciumoxids oder eines Aluminiumoxids, -hydroxids oder -oxyhydroxids ausgebildet ist.

8. Sol nach einem der vorangehenden Ansprüche, **dadurch gekennzeichnet, daß** die flüssige organische Phase auf Basis eines polaren Lösemittels ausgebildet ist.

9. Sol nach einem der vorangehenden Ansprüche, **dadurch gekennzeichnet, daß** die amphiphile Verbindung ausgewählt ist aus polyoxyethylenierten Alkyl- oder Alkylaryletherphosphaten.

10. Sol nach einem der vorangehenden Ansprüche, **dadurch gekennzeichnet, daß** das polare Lösemittel ausgewählt ist aus halogenierten Lösemitteln, Estern und Alkoholen.

11. Feste Verbindung, **dadurch gekennzeichnet, daß** sie eine Mischung von Titanoxidteilchen und mindestens einer amphiphilen Verbindung, ausgewählt aus polyoxyethylenierten Alkyletherphosphaten, umfaßt.

12. Feste Verbindung nach Anspruch 11, **dadurch gekennzeichnet, daß** die Titanoxidteilchen zumindest teilweise mit einer Schicht mindestens eines Metall- oder Siliciumoxids, -hydroxids oder -oxyhydroxids bedeckt sind.

13. Feste Verbindung nach Anspruch 11, **dadurch gekennzeichnet, daß** die Titanoxidteilchen zumindest teilweise bedeckt sind:

   - mit einer ersten Schicht mindestens einer Cer- und/oder Eisenverbindung und
   - mit einer zweiten Schicht mindestens eines Metall- oder Siliciumoxids, -hydroxids oder -oxyhydroxids.

14. Verfahren zur Herstellung eines Sols nach einem der Ansprüche 1 bis 10, **dadurch gekennzeichnet, daß** man die vorgenannte amphiphile Verbindung und die flüssige organische Phase mischt, dann die Titanoxidteilchen, gegebenenfalls bedeckt mit der oder den zwei vorgenannten Schichten, in der erhaltenen Mischung dispergiert.

**15.** Verfahren zur Herstellung eines Sols nach einem der Ansprüche 1 bis 10, **dadurch gekennzeichnet, daß** man eine Mischung von Titanoxidteilchen, gegebenenfalls bedeckt mit der oder den zwei vorgenannten Schichten, und mindestens einer vorgenannten amphiphilen Verbindung herstellt, dann diese Mischung in der flüssigen organischen Phase dispergiert.

**16.** Verfahren zur Herstellung eines Sols nach einem der Ansprüche 1 bis 10, umfassend eine flüssige organische Phase (a), insbesondere eines Sols in einer organischen Phase (a) auf Basis eines polaren Lösemittels, **dadurch gekennzeichnet, daß** man eine Dispersion, enthaltend Titanoxidteilchen, gegebenenfalls bedeckt mit der oder den vorgenannten zwei Schichten, und mindestens eine vorgenannte amphiphile Verbindung in einer flüssigen organischen Phase (b) auf Basis eines Lösemittels mit geringerer Polarität als die des Lösemittels der flüssigen organischen Phase (a), bildet; man die feste Phase von der flüssigen Phase (b) abtrennt; man die auf diese Weise erhaltene feste Phase in der organischen Phase (a) dispergiert.

**17.** Verfahren zur Herstellung eines Sols nach einem der Ansprüche 14 bis 16, **dadurch gekennzeichnet, daß** man als Ausgangsprodukt Titandioxidteilchen verwendet, die erhalten sind durch Hydrolyse mindestens einer Titanverbindung A in Gegenwart mindestens einer Verbindung B, ausgewählt aus

(i) Säuren, die aufweisen:

- entweder eine Carbobylgruppe und mindestens zwei Hydroxylund/oder Amingruppen,
- oder mindestens zwei Carboxylgruppen und mindestens eine Hydroxyl- und/oder Amingruppe.

(ii) organischen Phosphorsäuren der folgenden Formeln:

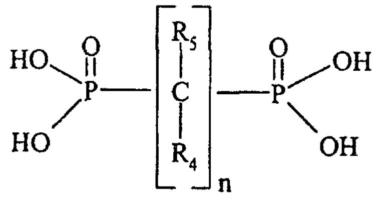

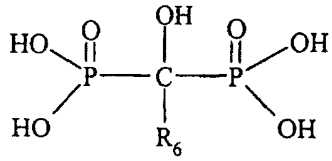

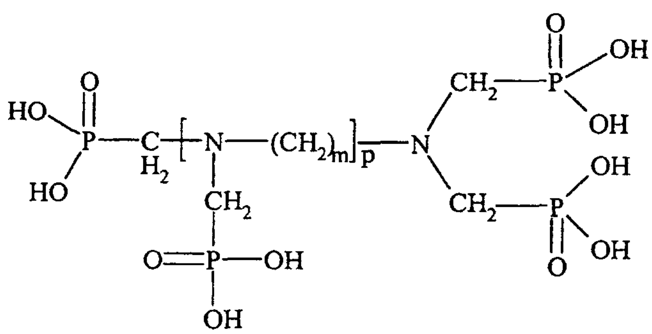

wobei n und m ganze Zahlen zwischen 1 und 6 sind, p eine ganze Zahl zwischen 0 und 5 ist und $R_4$, $R_5$ und $R_6$, identisch oder verschieden, eine Hydroxyl-, Amino-, Aralkyl-, Aryl- oder Alkylgruppe oder Wasserstoff darstellen,

(iii) Verbindungen, die in saurem Milieu Sulfationen freisetzen können,

(iv) Salzen der zuvor beschriebenen Säuren,

und in Gegenwart von Titandioxidkeimen vom Typ Anatas; und
anschließende Abtrennung des gebildeten Niederschlags vom Hydrolysemilieu.

**18.** Verfahren nach Anspruch 17, **dadurch gekennzeichnet, daß** man als Ausgangsprodukt Titandioxidteilchen verwendet, die erhalten sind durch das vorgenannte Hydrolyseverfahren, wobei die Titandioxidkeime vom Typ Anatas eine Teilchengröße von höchstens 8 nm aufweisen und in einem Gewichtsverhältnis, berechnet als $TiO_2$ in den Keimen/Titan vor Zugabe der Keime zu dem Hydrolysemilieu, berechnet als $TiO_2$, zwischen 0,01 % und 3 % aufweisen.

**19.** Verfahren nach Anspruch 17 oder 18, **dadurch gekennzeichnet, daß** man als Ausgangsprodukt Titandioxidteilchen verwendet, die erhalten sind durch das vorgenannte Hydrolyseverfahren, wobei die Titanverbindung A Titanoxychlorid ist.

**20.** Verfahren nach einem der Ansprüche 17 bis 19, **dadurch gekennzeichnet, daß** man als Ausgangsprodukt Titandioxidteilchen verwendet, die erhalten sind durch das vorgenannte Hydrolyseverfahren, wobei die Verbindung B Zitronensäure ist.

**21.** Verfahren nach einem der Ansprüche 17 bis 20, **dadurch gekennzeichnet, daß** man als Ausgangsprodukt Titandioxidteilchen verwendet, die erhalten sind durch ein Verfahren, welches die vorgenannte Hydrolyse umfaßt, wobei der gebildete Niederschlag vom Hydrolysemilieu abgetrennt wird und anschließend in Wasser in Dispersion gebracht wird, so daß man eine Titanoxiddispersion in Wasser erhält, wobei man diese Dispersion bei einer Temperatur von höchstens 120 °C trocknet.

**22.** Verfahren nach einem der Ansprüche 14 bis 21, **dadurch gekennzeichnet, daß** man das Sol einer Ultrafiltrationsbehandlung unterwirft.

**23.** Verwendung eines Sols nach einem der Ansprüche 1 bis 10 zur Herstellung von Formulierungen bzw. Rezepturen für kosmetische Zwecke, Lacke, Farben und in Kunststoffen.

## Claims

**1.** Organic sol, **characterized in that** it comprises:

- titanium oxide particles;
- an organic liquid phase;
- at least one amphiphilic compound chosen from the polyoxyethylenated alkyl ether phosphates.

**2.** Sol according to claim 1, **characterized in that** the titanium oxide particles are at least partially covered by a layer of at least one silicon or metallic oxide, hydroxide or oxyhydroxide.

**3.** Sol according to claim 1, **characterized in that** the titanium oxide particles are at least partially covered:

- by a first layer of at least one cerium and/or iron compound, and
- by a second layer of at least one silicon or metallic oxide, hydroxide or oxyhydroxide.

**4.** Sol according to claim 2 or 3, **characterized in that** the titanium oxide particles have a BET specific surface area of at least 70 $m^2/g$.

**5.** Sol according to one of claims 2 to 4, **characterized in that** the ratio by weight of the silicon or metallic oxide(s), hydroxide(s) or oxyhydroxide(s) to titanium dioxide is at most 60% by weight.

**6.** Sol according to one of claims 3 to 5, **characterized in that** the first aforementioned layer is based on at least one cerium compound with a content such that the ratio by weight of the cerium compound, expressed in Ce02, to the

titanium dioxide is at most 6% by weight.

**7.** Sol according to one of claims 2 to 6, **characterized in that** the aforementioned layer or the aforementioned second layer is based on silica and/or aluminium oxide, hydroxide or oxyhydroxide.

**8.** Sol according to one of the previous claims, **characterized in that** the organic liquid phase is based on a polar solvent.

**9.** Sol according to one of the previous claims, **characterized in that** the amphiphilic compound is chosen from polyoxyethylenated alkyl or alkylaryl ether phosphates.

**10.** Sol according to one of the previous claims, **characterized in that** the polar solvent is chosen from halogenated solvents, esters, alcohols.

**11.** Solid compound, **characterized in that** it comprises a mixture of titanium oxide particles and at least one amphiphilic compound chosen from polyoxyethylenated alkyl ether phosphates.

**12.** Sold compound according to claim 11, **characterized in that** the titanium oxide particles are at least partially covered with a layer of at least one silicon or metallic oxide, hydroxide or oxyhydroxide.

**13.** Solid compound according to claim 11, **characterized in that** the titanium oxide particles are at least partially covered:

- by a first layer of at least one cerium and/or iron compound, and
- by a second layer of at least one silicon or metallic oxide, hydroxide or oxyhydroxide.

**14.** Process for the preparation of a sol according to one of claims 1 to 10, **characterized in that** the aforementioned amphiphilic compound and the organic liquid phase are mixed together, then the titanium oxide particles, optionally covered with one or both of the aforementioned layers, are dispersed in the mixture obtained.

**15.** Process for the preparation of a sol according to one of claims 1 to 10, **characterized in that** a mixture is formed of titanium oxide particles, optionally covered by one or both of the aforementioned layers, and at least one aforementioned amphiphilic compound, then said mixture is dispersed in the liquid phase.

**16.** Process for the preparation of a sol according to one of claims 1 to 10 comprising an organic liquid phase (a), in particular a sol in an organic phase (a) based on a polar solvent, **characterized in that** a dispersion is formed comprising titanium oxide particles, optionally covered by one or both of the aforementioned layers, and at least one aforementioned amphiphilic compound in an organic liquid phase (b) based on a solvent with a lower polarity than that of the solvent of the organic liquid phase (a); the solid phase is separated from the liquid phase (b); the solid phase obtained in this way is dispersed in the organic phase (a).

**17.** Preparation process according to one of claims 14 to 16, **characterized in that**, as starting product, titanium dioxide particles are used which were obtained by hydrolysis of at least one titanium compound A in the presence of at least one compound B chosen from:

(i) acids which have:

- either a carboxyl group and at least two hydroxyl and/or amine groups,
- or at least two carboxyl groups and at least one hydroxyl and/or amine group,

(ii) organic phosphoric acids of the following formulae:

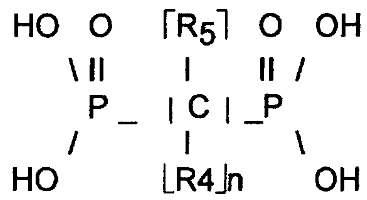

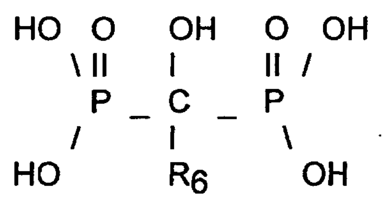

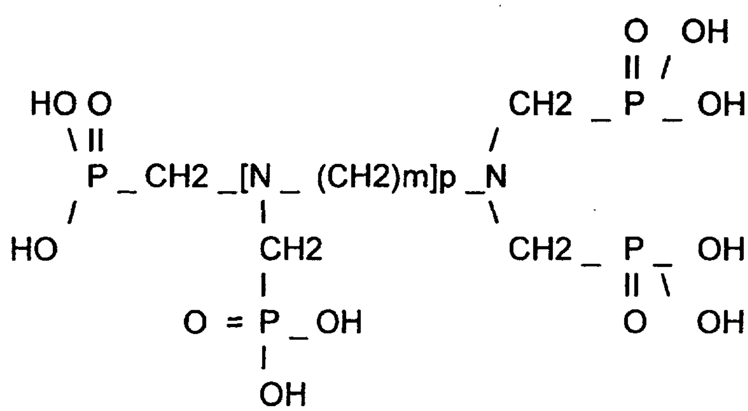

in which n and m are integers comprised between 1 and 6, p is an integer comprised between 0 and 5, $R_4$, $R_5$ and $R_6$ identical or different represent a hydroxyl, amino, aralkyl, aryl, alkyl group or hydrogen group,

(iii) the compounds capable of releasing sulphate ions in an acid medium,

(iv) salts of the acids described above

and in the presence of anatase titanium dioxide seeds;
then separation of the precipitate formed from the hydrolysis medium.

**18.** Process according to claim 17, **characterized in that**, as starting product, titanium dioxide particles are used which were obtained by the aforementioned hydrolysis process and in which the anatase titanium dioxide seeds are of a size no greater than 8 nm and are present in a ratio by weight expressed in TiO2 present in the seeds/titanium present before the introduction of the seeds into the hydrolysis medium, expressed in TiO2 comprised between 0.01% and 3%.

**19.** Process according to claim 17 or 18, **characterized in that**, as starting product, titanium dioxide particles are used which were obtained by the aforementioned hydrolysis process and in which the titanium compound A is titanium oxychloride.

**20.** Process according to one of claims 17 to 19, **characterized in that**, as starting product, titanium dioxide particles are used which were obtained by the aforementioned hydrolysis process and in which compound B is citric acid.

**21.** Process according to one of claims 17 to 20, **characterized in that**, as starting product, titanium dioxide particles are used which were obtained by a process comprising the aforementioned hydrolysis and in which the precipitate

formed is separated from the hydrolysis medium then redispersed in water resulting in a dispersion of titanium oxide in water and where said dispersion is dried at a temperature no greater than 120°C.

**22.** Process according to one of claims 14 to 21, **characterized in that** the sol is subjected to an ultrafiltration treatment.

**23.** Use of a sol according to one of claims 1 to 10 for the preparation of formulations for cosmetics, varnishes, paints and in plastics.